(19) Europäisches Patentamt
European Patent Office
Office européen des brevets

(11) Veröffentlichungsnummer: **0 061 029**
**B1**

(12) **EUROPÄISCHE PATENTSCHRIFT**

(45) Veröffentlichungstag der Patentschrift: **08.01.86**

(51) Int. Cl.[4]: **C 07 B 39/00**, C 07 C 17/14, C 07 C 21/24

(21) Anmeldenummer: **82101611.0**

(22) Anmeldetag: **03.03.82**

(54) **Verfahren zur Herstellung von Trichlormethyl-substituierten aromatischen Verbindungen.**

(30) Priorität: **14.03.81 DE 3109966**

(43) Veröffentlichungstag der Anmeldung: **29.09.82 Patentblatt 82/39**

(45) Bekanntmachung des Hinweises auf die Patenterteilung: **08.01.86 Patentblatt 86/2**

(84) Benannte Vertragsstaaten: **CH DE FR GB IT LI**

(56) Entgegenhaltungen:
**"Chemische Berichte", Band 98, 1965, VERLAG CHEMIE, Weinheim/Bergstrasse, R. MAYER et al. "Synthese einiger aromatischer Thiosäurechloride und Benzotrichloride", Seiten 829-837**
**Houben-Weyl, Methoden der organischen Chemie, Bd. V/3 (4. Aufl. 1962), S. 642-645 und S. 752-753**

(73) Patentinhaber: **BAYER AG, Konzernverwaltung RP Patentabteilung, D-5090 Leverkusen 1 Bayerwerk (DE)**

(72) Erfinder: **Marhold, Albrecht, Dr.,** ***Carl-Duisberg-Strasse 329*, D-5090 Leverkusen 1 (DE)**

**EP 0 061 029 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Trichlormethyl-substituierten aromatischen Verbindungen.

Es ist bekannt, Trichlormethyl-substituierte aromatische Verbindungen durch Seitenkettenchlorierung von Methyl-substituierten aromatischen Verbindungen herzustellen. So kann beispielsweise Benzotrichlorid durch Seitenkettenchlorierung von Toluol hergestellt werden (Houben-Weyl-Müller V, Band 3, 4. Auflage, 1962, Seite 738).

Aus Houben-Weyl, Band V/3 (1962), Seiten 642 bis 645 und 752 bis 753 sind Chlorierungen von Thioethern bekannt, bei denen an Schwefel gebundene Methylgruppen trichloriert oder an Schwefel gebundene Methylengruppen monochloriert werden, ausserdem die Spaltung von schwefelhaltigen organischen Verbindungen, insbesondere von Dialkylsulfiden und Dialkylpolysulfiden, wobei die Art der Spaltungsprodukte sehr vielfältig und undefiniert ist.

Methyl-substituierte aromtische Verbindungen, die noch andere Substituenten, beispielsweise Alkylgruppen, Alkoxygruppen, Halogene und Nitrogruppen enthalten, können so nicht zu Trichlormethyl-substituierten aromatischen Verbindungen chloriert werden, weil diese Substituenten entweder selbst mit Chlor reagieren oder eine Chlorierung der Methylgruppe verhindern. So kann beispielsweise Methylbenzotrichlorid nur durch Chlorierung von Methylbenzothiocarbonsäurechlorid hergestellt werden (R. Meyer, S. Scheitauer, Chem. Ber. Band 98, Seite 829).

Diese Umsetzung hat den Nachteil, dass die dabei benötigten Thiocarbonsäurechloride nur über eine Grignard-Reaktion zugänglich sind, die für die Herstellung grösserer Mengen nicht geeignet ist.

Es wurde ein Verfahren zur Herstellung von Trichlormethyl-substituierten aromatischen Verbindungen der Formel

$$Ar\text{–}CCl_3, \qquad (I)$$

in der

Ar für einen gegebenenfalls substituierten Arylrest steht, gefunden, das dadurch gekennzeichnet ist, dass Thioether der Formel

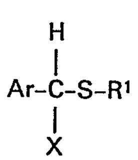

(II)

in der

Ar die oben angegebene Bedeutung hat,

X Wasserstoff oder Halogen bedeutet, und

$R^1$ für einen gegebenenfalls substituierten Arylrest, die Gruppe

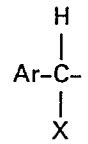

worin Ar und X die oben angegebene Bedeutung haben, gegebenenfalls substituiertes Alkyl, Aralkyl oder die Nitrilgruppe steht, gegebenenfalls in einem Lösungsmittel in dem Temperaturbereich von –20 bis 125°C mit 2 bis 12 Mol Chlor oder Sulfurylchlorid als Chlorierungsmittel pro Mol Thioether umgesetzt werden.

Als Aryl seien beispielsweise Reste mit 6 bis 14 C-Atomen wie Phenyl, Naphthyl, Anthryl genannt. Bevorzugt sind der Phenyl- und Naphthylrest.

Als Alkyl seien beispielsweise geradkettig oder verzweigte Reste mit 1 bis etwa 8 C-Atomen, bevorzugt ein Niederalkylrest (1 bis etwa 4 Kohlenstoffatome), wie Methyl, Ethyl, Propyl, Isopropyl, Butyl, Isobutyl, Hexyl, Octyl, Isooctyl, genannt. Bevorzugt sind Methyl, Ethyl, Propyl, Isopropyl, Butyl und Hexyl, besonders bevorzugt Methyl, Ethyl, Propyl und Butyl.

Als Aralkyl seien besipielsweise Reste mit 6 bis 14 C-Atomen im Arylteil und 1 bis 4 C-Atomen im Alkylteil wie Benzyl, 1-Naphthylmethyl, 2-Naphthylmethyl, 1-Anthrylmethyl, 2-Anthrylmethyl, 9-Anthrylmethyl, Phenylethyl, 1-Naphthylethyl, 2-Naphthylethyl, 1-Anthrylethyl, 9-Anthrylethyl, Phenylpropyl, Phenylisopropyl, 1-Naphthylpropyl, 1-Naphthylisopropyl, Phenylbutyl, Phenyl-2-butyl, 1-Naphthyl-butyl, 1-Naphthyl-2-butyl, 2-Naphthyl-1-butyl, 2-Naphthyl-2-butyl, genannt. Bevorzugt sind Reste mit 6 bis 10 C-Atomen im Arylteil und 1 bis 4 Kohlenstoffatomen im Alkylteil, besonders bevorzugt der Benzyl- und der 1-Naphthylmethylrest.

Als Halogen seien Fluor, Chlor, Brom und Jod, bevorzugt Fluor, Chlor und Brom, besonders bevorzugt Chlor und Brom, genannt.

Die Alkyl-, Aryl- und Aralkylreste können durch einen oder mehrere, bevorzugt einen, zwei oder drei, besonders bevorzugt einen oder zwei, gleiche oder verschiedene Reste substituiert sein.

Als Substituenten der Alkyl-, Aryl- und Aralkylreste seien beispielsweise Halogene wie Fluor, Chlor und Brom, Isocyanatgruppen, Cyanogruppen, Nitrogruppen, Carbonestergruppen mit 1 bis 4 C-Atomen im Alkoxyteil, Alkoxygruppen mit 1 bis 4 C-Atomen wie Methoxy, Ethoxy, Propoxy und Butoxy, Alkylgruppen mit 1 bis 4 C-Atomen wie Methyl, Ethyl, Propyl, Isopropyl, Butyl, 2-Butyl, tert.-Butyl, Aralkylgruppen wie Benzyl und Naphthyl und Arylgruppen wie Phenyl und Naphthyl genannt. Bevorzugt sind Chlor, Brom, Nitrogruppen, Carbonestergruppen, Alkoxygruppen wie Methoxy und Ethoxy, Alkylgruppen, der Benzylrest und die Phenylgruppe. Besonders bevorzugte Substituenten sind Chlor, Brom, Nitro und Methyl.

Bevorzugte Thioether für das erfindungsgemässe Verfahren sind Verbindungen der Formel

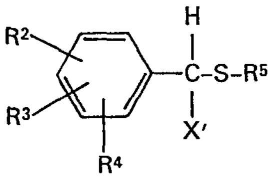

(III)

in der

$R^2$, $R^3$ und $R^4$ gleich oder verschieden sind und Wasserstoff, Halogen, Niederalkyl, Phenyl, Benzyl, Phenoxy oder Nitro bedeuten und wobei benachbarte Reste zu einem sechsgliedrigen Kohlenwasserstoffring verknüpft sein können,

X' Wasserstoff, Chlor oder Brom bedeutet und

$R^5$ einen gegebenenfalls durch Halogen, Niederalkyl oder Nitro substituierten Phenyl- oder Benzylrest, die Nitrilgruppe oder die Gruppe

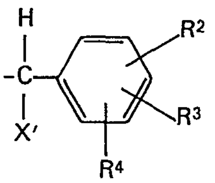

worin

$R^2$, $R^3$, $R^4$ und X' die oben angegebene Bedeutung haben, bedeuten.

Für das erfindungsgemässe Verfahren werden insbesondere Thioether der Formel

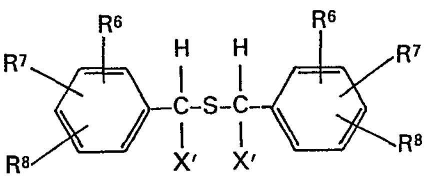

(IV)

in der

$R^6$, $R^7$ und $R^8$ gleich oder verschieden sind und Wasserstoff, Fluor, Chlor, Brom, Methyl, Ethyl, Propyl, Phenyl, Benzyl, Phenoxy oder Nitro bedeuten und

X' die oben angegebene Bedeutung hat, bevorzugt.

Im erfindungsgemässen Verfahren können beispielsweise folgende Thioether eingesetzt werden:

Benzylsulfid, o,m,p-Xylylsulfid, o,m,p-Brombenzylsulfid, o,m,p-Nitrobenzylsulfid, o,m,p-Ethylbenzylsulfid, o,m,p-Phenoxy-benzylsulfid, α,β-Naphthylmethylsulfid, o,m,p-Xylylrhodanid, o,m,p-Brombenzylrhodanid, o,m,p-Nitrobenzylrhodanid, o,m,p-Phenoxy-benzylrhodanid, o,m,p-Xylyl-phenyl-thioether, o,m,p-Brombenzyl-phenylthioether, o,m,p-Nitrobenzyl-phenylthioether, o,m,p-Xylyl-4-chlorphenyl-thioether, o,m,p-Brombenzyl-4-chlorphenyl-thioether, 2-Chlor-3-methyl-benzylsulfid und 4-Nitro-3-chlorbenzylsulfid.

Bevorzugt sind o,m,p-Xylylsulfid, o,m,p-Brombenzylsulfid, o,m,p-Nitrobenzylsulfid, o,m,p-Phenoxy-benzylsulfid, α,β-Naphthylmethylsulfid, o,m,p-Xylylrhodanid, o,m,p-Brombenzylrhodanid, o,m,p-Nitrobenzylrhodanid, o,m,p-Xylylphenyl-thioether, o,m,p-Brombenzyl-phenylthioether, o,m,p-Xylyl-4-chlorphenyl-thioether und 2-Chlor-3-methyl-benzylsulfid.

Die im erfindungsgemässen Verfahren eingesetzten Thioether, in denen der Rest X für Wasserstoff steht, können beispielsweise durch Umsetzung von Chlormethyl-substituierten aromatischen Verbindungen mit Natriumsulfid hergestellt werden (Houben-Weyl, 4. Auflage 1962, IX, Seite 97). Nach dieser Methode werden bevorzugt symmetrische Thioether hergestellt.

Im erfindungsgemässen Verfahren einsetzbare Thioether, in denen der Rest X für Wasserstoff steht, können auch durch Umsetzung von Chlormethyl-substituierten aromatischen Verbindungen mit Alkalisalzen von organischen Mercaptanen hergestellt werden (Houben-Weyl, 4. Auflage 1962, IX, Seite 103). Mit dieser Methode können symmetrische und unsymmetrische Thioether hergestellt werden.

Im erfindungsgemässen Verfahren einsetzbare Thioether, in denen der Rest X für Halogen steht, können nach Journal of American Chemical Society 89, 4483 (1967) durch Umsetzung von aromatischen Aldehyden mit organischen Mercaptanen in Gegenwart von Halogenwasserstoff hergestellt werden.

Im erfindungsgemässen Verfahren können auch unsymmetrische Thioether eingesetzt werden, so dass zwei verschiedene Trichlormethyl-substituierte aromatische Verbindungen gleichzeitig hergestellt werden können.

Als Chlorierungsmittel können im erfindungsgemässen Verfahren Chlor oder Sulfurylchlorid eingesetzt werden. Es können Mischungen dieser Chlorierungsmittel eingesetzt werden. Bevorzugt wird Chlor eingesetzt.

Pro Mol des Thioethers werden für jede zu chlorierende Gruppe 2 bis 12 Mol, bevorzugt 2 bis 8 Mol, besonders bevorzugt 2 bis 5 Mol Chlorierungsmittel eingesetzt.

Die Chlorierung nach dem erfindungsgemässen Verfahren kann durch an sich übliche katalytische Methoden zum Einleiten von radikalischen Reaktionen durchgeführt werden. So kann es vorteilhaft sein, das erfindungsgemässe Verfahren in Gegenwart von UV-Licht durchzuführen. Die Chlorierung kann auch mit Hilfe von üblichen organischen Radikalstartern wie Peroxiden, z.B. Benzoylperoxid, und Azoverbindungen, z.B. Azobisisobuttersäuredinitril, durchgeführt werden, wobei auch gegebenenfalls zusätzlich eine Bestrahlung mit UV-Licht erfolgen kann.

Falls das erfindungsgemässe Verfahren in Gegenwart eines Katalysators bzw. Radikalstarters durchgeführt wird, setzt man diese im allgemeinen in einer Menge von 0,1 bis 5 Gew.%, bezogen auf das gesamte Reaktionsgemisch, zu.

Bevorzugt werden organische Radikalstarter und Licht, besonders bevorzugt UV-Licht, eingesetzt. In einer bevorzugten Ausführungsform wird das erfindungsgemässe Verfahren mit grosser Selektivität ohne Katalysator ausgeführt.

Das erfindungsgemässe Verfahren kann mit und ohne Lösungsmittel durchgeführt werden. Als Lösungsmittel können inerte organische Lösungsmittel eingesetzt werden. Als inerte organische Lösungsmittel können beispielsweise halogenierte Lösungsmittel wie chlorierte Kohlenwasserstoffe, z.B. Chloroform, Tetrachlorkohlenstoff, Chlorbenzol, Tetrachlorethylen, Dichlorbenzol, fluorierte organische Lösungsmittel, beispielsweise Benzotrifluorid und Oxihalogenide von Elementen der 5. Hauptgruppe, beispielsweise Oxihalogenide des Phosphors wie Phosphoroxichlorid, eingesetzt werden.

Bevorzugt werden chlorierte Kohlenwasserstoffe, wie Tetrachlorkohlenstoff, Chloroform, Chlorbenzol und Oxichloride von Elementen der 5. Hauptgruppe, wie Oxichloride des Phosphors, eingesetzt. Besonders bevorzugt werden Tetrachlorkohlenstoff und Phosphoroxichlorid eingesetzt.

Pro Mol Thioether werden 50 bis 2000 ml, bevorzugt 100 bis 1000 ml, besonders bevorzugt 100 bis 500 ml Lösungsmittel eingesetzt.

Das erfindungsgemässe Verfahren kann auch in Lösungsmittelgemischen durchgeführt werden.

Die Ausführung des erfindungsgemässen Verfahrens kann bevorzugt dann ohne Lösungsmittel erfolgen, wenn der eingesetzte Thioether bei der gewählten Umsetzungstemperatur flüssig ist.

Das erfindungsgemässe Verfahren wird im Temperaturbereich von -20 bis 125°C, bevorzugt 10 bis 110°C, besonders bevorzugt 20 bis 100°C, durchgeführt.

Das erfindungsgemässe Verfahren kann beispielsweise so durchgeführt werden, dass der Thioether und gegebenenfalls der Katalysator vorgelegt werden. Dazu wird das Chlorierungsmittel gegeben.

Das erfindungsgemässe Verfahren kann auch so durchgeführt werden, dass das Chlorierungsmittel vorgelegt wird. Dazu wird der Thioether, gegebenenfalls vermischt mit einem Katalysator, gegeben.

Das erfindungsgemässe Verfahren kann auch so durchgeführt werden, dass das Chlorierungsmittel und gegebenenfalls der Katalysator vorgelegt werden. Dazu wird der Thioether gegeben.

Eine weitere mögliche Ausführungsform des erfindungsgemässen Verfahrens besteht darin, dass der Katalysator vorgelegt wird. Dazu wird simultan der einzusetzende Thioether und das Chlorierungsmittel gegeben.

Das erfindungsgemässe Verfahren kann weiterhin so durchgeführt werden, dass der Thioether vorgelegt wird. Dabei wird der Katalysator während der Zugabe von Chlorierungsmittel zugegeben.

Bevorzugt wird das erfindungsgemässe Verfahren so ausgeführt, dass zum vorgelegten Thioether das Chlorierungsmittel gegeben wird.

Bei einer Ausführung des erfindungsgemässen Verfahrens können vor Beginn der Umsetzung die Reaktionsteilnehmer mit Chlorwasserstoffgas gesättigt werden.

Das erfindungsgemässe Verfahren kann wie folgt ausgeführt werden:

Der Thioether wird gegebenenfalls in einem Lösungsmittel auf die Umsetzungstemperatur erhitzt. Dann wird so lange Chlorierungsmittel zugegeben, bis kein Chlor mehr aufgenommen wird, was am Aufhören der HCl-Entwicklung erkannt werden kann. Nach Abkühlen auf Raumtemperatur wird das Lösungsmittel und entstandenes Schwefelchlorid abdestilliert. Das als Rückstand verbleibende Umsetzungsprodukt wird durch Destillation und/oder Kristallisation gereinigt. Entstehen bei der Durchführung des erfindungsgemässen Verfahrens zwei verschiedene Trichlormethyl-substituierte aromatische Verbindungen, so können diese durch Kristallisation und/oder Destillation gereinigt und gegebenenfalls getrennt werden.

Trichlormethyl-substituierte aromatische Verbindungen nach dem erfindungsgemässen Verfahren können durch Fluorierung mit Fluorwasserstoff in Trifluormethyl-substituierte aromatische Verbindungen übergeführt werden, die Zwischenprodukte für die Synthese von Pflanzenschutzmitteln sind (DE-OS 2 537 753).

Mit Hilfe des erfindungsgemässen Verfahrens ist es überraschenderweise möglich, durch die bekannten Verfahren bisher nicht oder nur schwierig herstellbare trichlormethylsubstituierte aromatische Verbindungen herzustellen. So können beispielsweise trichlormethylsubstituierte aromatische Verbindungen der Formel

$$Ar^1\text{–}CCl_3, \qquad (V)$$

in der

$Ar^1$ für einen Bromphenyl- oder einen Naphthylrest steht, der durch Alkyl oder Alkoxy mit mehr als 2 Kohlenstoffatomen, Aryl, Aralkyl, oder Halogen substituiert ist, hergestellt werden.

Im allgemeinen sind die trichlormethylsubstituierten aromatischen Verbindungen der Formel (V) mit 1 bis 3, bevorzugt 1 oder 2 Resten substituiert. Bromphenyl ist o-, m- oder p-Bromphenyl, bevorzugt m-Bromphenyl.

Bevorzugt werden trichlormethylsubstituierte Verbindungen der Formel

$$Ar^2\text{–}CCl_3, \qquad (VI)$$

in der

$Ar^2$ für einen Bromphenyl- oder einen Naphthylrest steht, der durch geradkettiges oder verzweigtes Alkyl oder Alkoxy mit 2 bis 8 Kohlenstoffatomen, Phenyl, Benzyl, Fluor, Chlor oder Brom substituiert ist, hergestellt.

Im einzelnen seien beispielsweise die folgenden trichlormethylsubstituierten aromatischen Verbindungen genannt: o-Brombenzotrichlorid, p-Brombenzotrichlorid, 2,4-Dibrombenzotrichlorid, 2-Brom-3-methylbenzotrichlorid, 3-Brom-4-methylbenzotrichlorid, 3-Nitro-4-Methylbenzotrichlorid, 4-Nitro-3-methylbenzotrichlorid,

o,m,p-Ethylbenzotrichlorid, o,m,p-Phenylbenzotrichlorid und o,m,p-Benzylbenzotrichlorid.

Die Benzotrichloride können durch Verseifung in Gegenwart von Schwefelsäure [Houben-Weyl Bd. 8, Seiten 426 ff (1962)] in entsprechende Carbonsäuren überführt werden. Hierbei führen diese Benzotrichloride zu hochwirksamen Insektiziden (DE-OS 2 926 987), die sonst nur über mehrere Reaktionsschritte erhältlich sind.

Beispiel 1

100 g m-Xylylsulfid werden in 100 ml Tetrachlorkohlenstoff gelöst. Nach Erhitzen auf 80°C wird so lange Chlor eingeleitet, bis kein Chlor mehr aufgenommen wird. Das Lösungsmittel und entstandenes Schwefelchlorid werden im Vakuum abdestilliert. Als Rückstand verbleiben 164 g Reaktionsprodukt, das zu 55,2 Gew.% (bestimmt durch GC-Analyse) aus 3-Methylbenzotrichlorid besteht. Durch Destillation über eine Kolonne wird reines 3-Methylbenzotrichlorid vom Kp: 108 bis 109°C/12 mm, $n_D^{20}$ : 1,5545 erhalten.

Beispiel 2

300 g m-Xylylsulfid werden auf 90°C erhitzt und mit trockenem Chlorwasserstoffgas gesättigt. Anschliessend werden bei dieser Temperatur ca. 400 g Chlor eingeleitet. Zu Beginn der Umsetzung ist die Chloraufnahme quantitativ und erst gegen Ende entweicht Chlor aus dem Umsetzungsgemisch. Entstandenes Schwefelchlorid wird abdestilliert. Es verbleiben 477 g Rohprodukt, das destilliert wird. Das Destillat enthält 54,2 Gew.% 3-Methylbenzotrichlorid.

Beispiel 3

30 g m-Xylylsulfid werden in 150 ml Tetrachlorkohlenstoff gelöst. Bei 20 bis 30°C wird Chlor bis zur Sättigung eingeleitet. Das nach der Beendigung der Chloreinleitung erhaltene Umsetzungsgemisch enthält 51,3 Gew.% (bestimmt durch GC-Analyse) 3-Methylbenzotrichlorid.

Beispiel 4

30 g m-Xylylsulfid werden in 80 ml Phosphoroxichlorid gelöst. Nach Erhitzen auf 70 bis 75°C wird Chlor bis zur Sättigung eingeleitet. Nach Abdestillieren des Lösungsmittels und entstandenen Schwefelchlorids werden nach Destillation des erhaltenen Reaktionsgemisches 20 g 3-Methylbenzotrichlorid isoliert.

Beispiel 5

Beispiel 4 wird mit Chlorbenzol anstelle von Phosphoroxichlorid als Lösungsmittel wiederholt. Es werden 22,8 g 3-Methylbenzotrichlorid erhalten.

Beispiel 6

520 g p-Xylylsulfid werden in 500 ml Tetrachlorkohlenstoff gelöst. Bei der Rückflusstemperatur der Lösung wird Chlor bis zu Sättigung eingeleitet. Nach Abdestillieren des Lösungsmittels und entstandenen Schwefelchlorids wird das erhaltene Umsetzungsprodukt destilliert. Es werden 430 g 4-Methylbenzotrichlorid vom Kp: 104 bis 105°C/9 mm erhalten, die bei 46°C zu Kristallen erstarren.

Beispiel 7

200 g o-Xylylsulfid werden wie in Beispiel 6 beschrieben umgesetzt. Es werden 105 g 2-Methylbenzotrichlorid vom Kp: 108 bis 110°C/11 mm, $n_D^{20}$ : 1,5655 erhalten.

Beispiel 8

100 g 1-Naphthylmethylsulfid werden mit 250 ml Tetrachlorkohlenstoff gelöst. Bei 75 bis 80°C wird bis zur Sättigung Chlor in die Lösung eingeleitet. Anschliessend wird das Lösungsmittel und das entstandene Schwefelchlorid abdestilliert. Aus dem als Rückstand verbliebenen Umsetzungsprodukt werden nach fraktionierter Destillation 30 g 1-Trichlormethylnaphthalin vom Kp: 124 bis 128°C/0,3 mm erhalten.

Beispiel 9

Bis-(4-nitrobenzyl)-sulfid werden in 300 ml Phosphoroxichlorid gelöst. Bei 70 bis 80°C werden 300 g Chlor innerhalb von 1 Stunde eingeleitet. Nach Abkühlen auf ca. 20°C wird nochmals 30 Minuten Chlor eingeleitet. Nach Abdestillieren des Schwefelchlorids wird das so erhaltene Rohprodukt durch Destillation gereinigt. Es werden 209 g 4-Nitrobenzotrichlorid vom Kp: 115 bis 118°C/0,1 mm erhalten, die bei 42 bis 44°C zu Kristallen erstarren.

Beispiel 10

20 g m-Xylylrhodanid werden in 100 ml Phosphoroxichlorid gelöst. Bei der Rückflusstemperatur der Lösung wird bis zur Sättigung Chlor eingeleitet (für ca. 8 Stunden). Nach Beendigung der Chloreinleitung wird durch GC-Analyse ein Gehalt an 50,5 Gew.% 3-Methylbenzotrichlorid festgestellt.

Beispiel 11

150 g 4-Chlorphenyl-(4-methyl)-benzyl-thioether werden in 200 ml Tetrachlorkohlenstoff gelöst. Bei 80°C wird Chlor bis zur Sättigung eingeleitet. Nach Beendigung der Umsetzung werden nach Destillation über eine Kolonne 85 g 4-Methylbenzotrichlorid erhalten.

Beispiel 12

48 g o-Xylylsulfid werden in 100 ml Tetrachlorkohlenstoff gelöst. Bei 60°C werden 200 g Sulfurylchlorid zugetropft. Nach Beendigung der Zugabe wird 5 Stunden bei dieser Temperatur nachgerührt. Nach destillativer Aufarbeitung werden 21 g 2-Methylbenzotrichlorid erhalten.

Beispiel 13

80 g 2-Brombenzyl-thioether werden in 200 ml Tetrachlorkohlenstoff gelöst. Bei 75°C wird bis zur Sättigung der Lösung Chlor eingeleitet. Nach Abkühlen auf 20°C wird nochmals für 30 Minuten Chlor eingeleitet. Anschliessend wird die Reaktionsmischung bei 0,5 mm destilliert. Man erhält bei einer Übergangstemperatur von 82 bis 88°C/0,5 mm 36 g 2-Brombenzotrichlorid (30% der Theorie).

Beispiel 14

121 g m-Xylylsulfid werden in 250 ml Tetrachlorkohlenstoff gelöst. Bei 20°C und gleichzeitiger Bestrahlung mit UV-Licht werden 220 g Chlor innerhalb 4 Stunden eingeleitet. Nach Abdestillieren des Lösungsmittels verbleiben 190 g Rohprodukt, das nach GC-Analyse 34 Gew.% 3-Methylbenzotrichlorid enthält.

Beispiel 15

50 g 4-Methoxy-α-chlorbenzyl-(4-chlorphenyl)-thioether werden in 50 ml Tetrachlorkohlenstoff gelöst. Bei 25°C wird mit der Chloreinleitung begonnen. Es wird Chlor bis zur Sättigung eingeleitet, wobei die Temperatrur des Umsetzungsgemisches während der Chloreinleitung auf 50°C ansteigt. Nach Beendigung der Chloraufnahme wird das Lösungsmittel abdestilliert und der Rückstand grob destilliert. Übergangstemperatur: 105 bis 165°C/20 mbar. Es werden 60 g Destillat erhalten, das nach GC-Analyse 17,4 Gew.% 4-Methoxybenzotrichlorid enthält.

Die folgenden Beispiele 16 bis 24 erläutern die Herstellung von Ausgangsverbindungen für das erfindungsgemässe Verfahren.

Beispiel 16

4-Chlorphenyl-(4-methyl)-benzyl-thioether

In einer Rührapparatur werden 150 g 4-Chlorthiophenol in 200 ml Wasser, 65 g Kaliumhydroxid, 10 g Tetraethylammoniumchlorid und 140 g 4-Methylbenzylchlorid vorgelegt und für 4 Stunden auf Rückflusstemperatur erhitzt. Dann werden 300 ml Wasser zugegeben und abgekühlt. Das Festprodukt wird abgesaugt und mit Wasser gewaschen. Nach Trocknen der Kristalle verbleiben 230 g Produkt vom Schmp. 72 bis 73°C.

Beispiel 17

4-Methoxy-α-chlorbenzyl-(4-chlorphenyl)-thioether

144 g 4-Chlorthiophenol werden in 300 ml Methylenchlorid vorgelegt und 136 g 4-Methoxybenzaldehyd in 300 ml Methylenchlorid zugegeben. Nach Zugabe von 80 g Calciumchlorid wird für 6 Stunden trockener Chlorwasserstoff eingeleitet. Nach Stehen über Nacht wird filtriert und die Methylenchloridlösung eingeengt. Das Festprodukt wird aus Petrolether umkristallisiert. Es werden 210 g Kristalle vom Schmp. 76 bis 77°C erhalten.

Beispiel 18

m-Xylylsulfid

In 900 ml Methanol werden 650 g 3-Methylbenzylchlorid bei Rückfluss vorgelegt und 585 g Natriumsulfid-monohydrat gelöst in 450 ml Wasser innerhalb 1 Stunde zugetropft. Nach Ende der Zugabe wird noch für 5 Stunden unter Rückfluss gerührt, dann abgekühlt und mit 2000 ml Wasser verdünnt. Die organische Phase wird mit Methylenchlorid abgetrennt und nach Trocknung destilliert. Es werden bei einer Übergangstemperatur von 142 bis 145°C/0,25 mm 495 g m-Xylylsulfid erhalten.

Beispiel 19

o-Xylylsulfid

Versuchsdurchführung wie in Beispiel 18. Ausbeute 92% (der Theorie) vom Schmp. 86 bis 88°C.

Beispiel 20

p-Xylylsulfid

Versuchsdurchführung wie in Beispiel 18. Ausbeute 97% (der Theorie) vom Schmp. 75 bis 76°C.

Beispiel 21

4-Nitrobenzylsulfid

Versuchsdurchführung wie in Beispiel 18. Ausbeute 94% (der Theorie) vom Schmp. 157 bis 159°C.

Beispiel 22

1-Naphthylmethylsulfid

Versuchsdurchführung wie im Beispiel 18. Es werden 88% (der Theorie) vom Schmp. 104 bis 106°C erhalten.

Beispiel 23

2-Brombenzylsulfid

Versuchsdurchführung wie im Beispiel 18. Es werden 94% (der Theorie) vom Schmp. 62 bis 63°C erhalten.

Beispiel 24

In 1000 ml Trichlormethan werden 595 g 3-Nitro-4-methylbenzylsulfid bei Rückflusstemperatur vorgelegt und bis Sättigung mit Chlor umgesetzt. Dann wird noch für 1 Stunde bei 20°C weiter Chlor eingeleitet und anschliessend mit Stickstoff ausgeblasen. Durch fraktionierte Destillation werden bei Kp: 128–130°C/2,5 mbar 315 g 3-Nitro-4-methylbenzotrichlorid erhalten.

Das eingesetzte 3-Nitro-4-methyl-benzylsulfid wurde analog Beispiel 21 aus 3-Nitro-4-methylbenzylchlorid erhalten.

Beispiel 25

Durch Einleiten von Chlor bis zur Sättigung in eine Lösung von 80 g 3-Methyl-4-nitro-benzylsulfid in 150 ml Trichlormethan bei Rückfluss werden 115 g rohes Reaktionsprodukt (nach Abdestillieren des Lösungsmittels) erhalten, das nach GC-Analyse zu 55,5% aus 3-Methyl-4-nitrobenzotrichlorid besteht.

Das eingesetzte 3-Methyl-4-nitro-benzylsulfid wurde analog Beispiel 21 aus 3-Nitro-4-methylbenzylchlorid erhalten.

Die folgenden Beispiele werden analog zu Beispiel 25 durchgeführt:

| Beispiel Nr. | Ausgangs-verbindung | Reaktions-produkt | Siedepunkt °C | Schmelzpunkt °C | Brechungs-index $n_D^{20}$ |
|---|---|---|---|---|---|
| 26 | 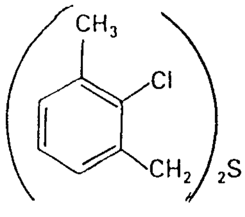 | 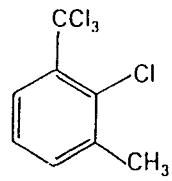 | 142–145 °C bei 16 mbar | 56–58 °C | |
| 27 | 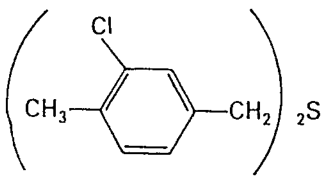 | 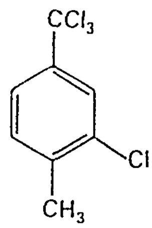 | 139–142 °C bei 20 mbar | 42–44 °C | |
| 28 | 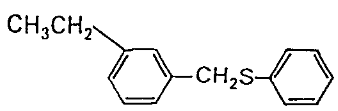 | 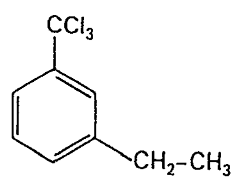 | 151–155 °C bei 12 mbar | | 1.5488 |
| 29 | 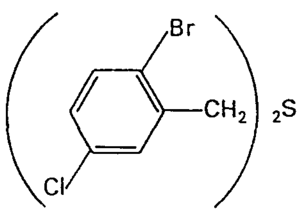 | 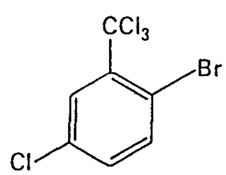 | 133–135 °C bei 0,4 mbar | | 1.6363 |
| 30 | 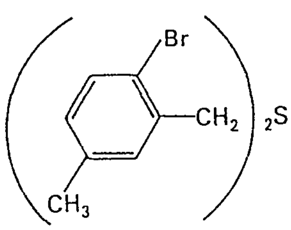 | 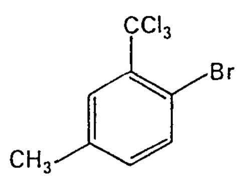 | 105–110 °C bei 0,2 mbar | | 1.6185 |
| 31 | 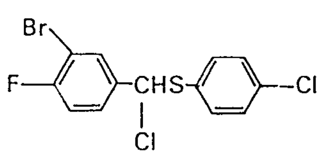 | 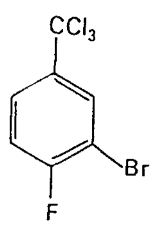 | 88–90 °C bei 1,5 mbar | | 1.5994 |
| 32 | 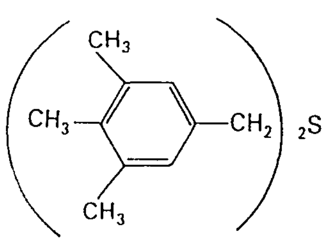 | 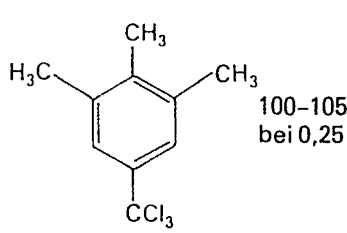 | 100–105 °C bei 0,25 mbar | | 1.5640 |

## Patentansprüche

1. Verfahren zur Herstellung von Trichlormethyl-substituierten aromatischen Verbindungen der Formel

$Ar\text{-}CCl_3$,

in der
Ar für einen gegebenenfalls substituierten Arylrest steht, dadurch gekennzeichnet, dass Thioether der Formel

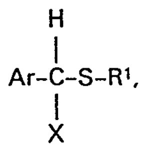

worin
Ar die oben angegebene Bedeutung hat,
X Wasserstoff oder Halogen bedeutet, und
$R^1$ für einen gegebenenfalls substituierten Arylrest, die Gruppe

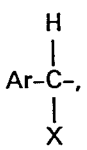

worin Ar und X die oben angegebene Bedeutung haben, gegebenenfalls substituiertes Alkyl, Aryl, Aralkyl und die Nitrilgruppe steht, gegebenenfalls in einem Lösungsmittel in dem Temperaturbereich von –20 bis 125°C mit 2 bis 12 Mol Chlor oder Sulfurylchlorid als Chlorierungsmittel pro Mol Thioether umgesetzt werden.

2. Verfahren nach Anspruch 1, dadurch gekennzeichnet, dass die Umsetzung ohne Lösungsmittel ausgeführt wird, wenn der eingesetzte Thioether bei der Umsetzungstemperatur flüssig ist.

3. Verfahren nach den Ansprüchen 1 und 2, dadurch gekennzeichnet, dass die Umsetzung in Gegenwart eines Katalysators ausgeführt wird.

4. Verfahren nach den Ansprüchen 1 bis 3, dadurch gekennzeichnet, dass die Umsetzung in Gegenwart eines organischen Radikalstarters und von UV-Licht durchgeführt wird.

5. Verfahren nach den Ansprüchen 1 bis 5, dadurch gekennzeichnet, dass Chlor verwendet wird.

6. Verfahren nach den Ansprüchen 1 bis 5, dadurch gekennzeichnet, dass Sulfurylchlorid verwendet wird.

7. Verfahren nach den Ansprüchen 1 bis 6, dadurch gekennzeichnet, dass vor Beginn der Umsetzung die Reaktionsteilnehmer mit Chlorwasserstoffgas gesättigt werden.

## Claims

1. Process for the preparation of trichlormethyl-substituted aromatic compounds of the formula

$Ar\text{-}CCl_3$,

in which
Ar represents an optionally substituted aryl radical, characterised in that thioethers of the formula

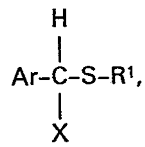

wherein
Ar has the meaning given above,
X denotes hydrogen or halogen, and
$R^1$ represents an optionally substituted aryl radical, the group

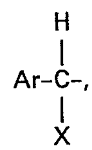

wherein
Ar and X have the meaning given above, optionally substituted alkyl, aryl, or aralkyl and the nitrile group, are reacted with 2 to 12 mols of chlorine or sulphuryl chloride, as the chlorinating agent, per mol of thioether, at a temperature in the range of –20 to 125°C, if appropriate in a solvent.

2. Process according to Claim 1, characterised in that the reaction is carried out without a solvent when the thioether used is liquid at the reaction temperature.

3. Process according to Claims 1 and 2, characterised in that the reaction is carried out in the presence of a catalyst.

4. Process according to Claims 1 to 3, characterised in that the reaction is carried out in the presence of an organic free radical initiator and UV light.

5. Process according to Claims 1 to 5, characterised in that chlorine is used.

6. Process according to Claims 1 to 5, characterised in that sulphuryl chloride is used.

7. Process according to Claims 1 to 6, characterised in that before the beginning of the reaction the reactants are saturated with hydrogen chloride gas.

## Revendications

1. Procédé de production de composés aromatiques à substituant trichlorométhyle, de formule

$Ar\text{-}CCl_3$,

dans laquelle
Ar représente un reste aryle éventuellement substitué, caractérisé en ce qu'on fait réagir des thioéthers de formule

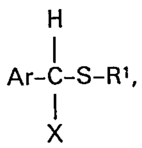

dans laquelle
Ar a la définition indiquée ci-dessus,
X désigne l'hydrogène ou un halogène et
$R^1$ est un reste aryle éventuellement substitué, le groupe

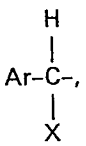

dans lequel Ar et X ont la définition indiquée ci-dessus, un groupe alkyle, aryle, aralkyle éventuellement substitué et le groupe nitrile, le cas échéant dans un solvant, dans l'intervalle de températures de −20 à 125°C, avec 2 à 12 moles de chlore ou de chlorure de sulfuryle comme agent de chloration par mole de thioéther.

2. Procédé suivant la revendication 1, caractérisé en ce qu'on conduit la réaction sans solvant lorsque le thioéther utilisé est liquide à la température de réaction.

3. Procédé suivant les revendications 1 et 2, caractérisé en ce qu'on conduit la réaction en présence d'un catalyseur.

4. Procédé suivant les revendications 1 à 3, caractérisé en ce qu'on conduit la réaction en présence d'un amorceur radicalaire organique et de lumière ultraviolette.

5. Procédé suivant les revendications 1 à 5, caractérisé en ce qu'on utilise du chlore.

6. Procédé suivant les revendications 1 à 5, caractérisé en ce qu'on utilise du chlorure de sulfuryle.

7. Procédé suivant les revendications 1 à 6, caractérisé en ce que les partenaires réactionnels sont saturés de chlorure d'hydrogène gazeux avant le début de la réaction.